Europäisches Patentamt

European Patent Office

Office européen des brevets

(19)

(11) Veröffentlichungsnummer: **0 351 766**
**A2**

(12) EUROPÄISCHE PATENTANMELDUNG

(21) Anmeldenummer: 89113114.6

(22) Anmeldetag: 18.07.89

(51) Int. Cl.⁴: **A61K 7/48**

(30) Priorität: 19.07.88 DE 3824370

(43) Veröffentlichungstag der Anmeldung:
24.01.90 Patentblatt 90/04

(84) Benannte Vertragsstaaten:
FR IT

(71) Anmelder: HARRO BOERNER MEDIZINISCHE
KOMMUNIKATION GMBH
Hedwig-Dransfeld-Strasse 12
D-6000 Frankfurt 90(DE)

(72) Erfinder: Dembowski, Elisabeth, Dr. med.
Marktplatz 12
D-3312 Salzgitter 51 (Bad)(DE)

(74) Vertreter: Patentanwälte Phys. Bartels
Dipl.-Ing. Fink Dr.-Ing. Held
Lange Strasse 51
D-7000 Stuttgart 1(DE)

(54) Dermatologisches Präparat.

(57) Ein dermatologisches Präparat mit Basiscreme und einem Konservierungsmittel enthält natürliche Sole.

EP 0 351 766 A2

## Dermatologisches Präparat

Gegenstand der Erfindung ist ein dermatologisches Präparat, das natürliche Sole enthält. Die natürliche Sole, deren Rezepturanteil vorzugsweise in der Größenordnung von 25 % liegt, ist ein idealer Wirkstoff, um Feuchtigkeit in der Hornschicht der Haut zu binden. Dabei ist von besonderem Vorteil, daß mit der natürlichen Sole ein komplexes Gemisch verschiedener Mineralstoffe und Spurenelemente zur Wirkung kommt. Die solebedingte Wasserbindung in der Haut fördert die Penetration auch der vorzugsweise vorhandenen übrigen Wirkstoffe und erhöht die Hautelastizität entscheidend. Der osmotische Effekt der Sole stimuliert den Gefäßtonus und die Hautdurchblutung. Das erfindungsgemäße Präparat hat deshalb ein breites Anwendungsspektrum von normaler Haut über beanspruchte Problemhaut bis zur therapiebedürftigen Haut. Es fördert bei einer leichten Erkrankung der Haut den Heilungsprozeß, vermag Hautkrankheiten zu verhindern und eignet sich zur Pflege, zur Behandlung kranker Haut und auch zur Nachbehandlung vormals kranker Haut. Das erfindungsgemäße Präparat vermag also feuchtigkeitsbindend, keratolythisch, keratoplastisch, entzündungshemmend, heilend, regenerierend, gefäßwandtonisierend, durchblutungsfördernd und penetrationsfördernd zu wirken.

Bei einer bevorzugten Ausführungsform des erfindungsgemäßen Präparates handelt es sich bei der Sole um solche der Solequelle Salzgitter-Bad.

Vorzugsweise enthält das Präparat wenigstens einen weiteren Wirkstoff. Besonders geeignete Wirkstoffe sind Panthenol, Harnstoff, Vitamine, Teer, Milchsäure, Collagen und Schwefel. Die weiteren Wirkstoffe üben Schutzwirkungen auf Epithelzellen aus. Aufgrund der antioxidativen Wirkung der Vitaminzusätze werden entzündliche Prozesse gehemmt und die Hautregeneration beschleunigt. Auch oxidative Umwelt-Aggressionen werden in ihrer Wirkung auf die Haut gemildert. Ferner enthält das erfindungsgemäße Präparat vorteilhafterweise Euxyl als Konservierungsmittel.

Weiterhin ist es vorteilhaft, wenn das Kosmetikum einen Zusatzemulgator enthält. Dabei kommt insbesondere Genapol, Hostapon und Texapon in Frage.

In vielen Fällen ist es vorteilhaft, wenigstens einen pflanzlichen Zusatz vorzusehen. Es kommen dabei Kräuter, wie beispielsweise Münzkraut, Augentrost, Heidekraut, Feldstiefmütterchen, Ackerschachtelhalm, Seifenwurzel, Aloe vera, Salbei, Ratanhia, Königskerze, Beinwell, Sonnenhut, Mannstreu in Frage, und zwar sowohl einzeln als auch in Form von Kombinationen.

Als Basiscreme ist die bekannte Basiscreme DAC sehr vorteilhaft, die folgende Rezeptur hat:

| | |
|---|---|
| Glycerolmonostearat | 4,0 |
| Cethylalkohol | 6,0 |
| Polyoxyäthylenglycerolmonostearat | 7,0 |
| Mittelkettige Triglyceride | 7,5 |
| Propylenglycol | 10,0 |
| weißes Vaselin | 25,5 |
| destilliertes Wasser | 40,0 |

Fettarme Varianten werden durch Zumischen von Wasser, fettere Varianten durch Zumischen von Vaseline erhalten, wodurch sich ein besonders breiter Anwendungsbereich des erfindungsgemäßen Präparates erreichen läßt.

Im folgenden ist die Erfindung anhand von Ausführungsbeispielen erläutert:

| 1. Rezeptur für eine Handcreme | |
|---|---|
| Vit.E | 0,2 |
| Vit.A 3000E/g | |
| Sole 25 % | 1,0 |
| Allantoin | 0,2 |
| Panthenol | 5,0 |
| Ac.lact. | 0,02 |
| Hydroviton | 5,0 |
| Aq. dest. | 75,0 |
| Cremor basal. | 100,0 |
| Parfümöl, cumarinfrei Q.s. | |
| Euxyl 400 Q.s. | |
| Als Zusatz eignet sich Ratanhia. | |

| 2. Rezeptur für eine Tagespflegecreme | |
|---|---|
| Urea pur | 2,0 |
| Ac. lact. | 0,02 |
| Allantoin | 0,2 |
| Sole 25 % | 3,0 |
| Vit. E | 0,2 |
| Vit. | 3 000E/g |
| Panthenol | 1,0 |
| Hydroviton | 5,0 |
| Vas. alb. | 30,0 |
| Cremor basalis | 100,0 |
| Euxyl 400 | q.s. |
| Parfümöl, cumarinfrei q.s. | |
| Als weiterer Zusatz ist Kastanie besonders vorteilhaft. | |

| 3. Rezeptur für eine Nachtpflegecreme | |
|---|---|
| Urea pur | 4,0 |
| Ac. lact. | 0.04 |
| Allantoin | 0,4 |
| Sole 25 % | 6,0 |
| Vit. E | 0,4 |
| Vit. A | 3 000E/g |
| Panthenol | 2,0 |
| Vas. alb | 100,0 |
| Cremor Basalis | 100,0 |
| Euxyl 400 | q.s. |
| Parfümöl, cumarinfrei q.s. | |
| Als Zusatz ist Münzkraut vorteilhaft. | |

| 4. Rezeptur für eine Tagespflegecreme für fettige, unreine Haut und Mischhaut | |
|---|---|
| Urea pur | 3,0 |
| Ac. lact. | 0.04 |
| Allantoin | 0,2 |
| Sole 25 % | 5,0 |
| Vit. E | 0,2 |
| Vit. B6 | 0,2 |
| Vit. A | 5 000E/g |
| Panthenol | 1,0 |
| Hydroviton | 5,0 |
| Aq. dest. | 30,0 |
| Cremor basalis | 100,0 |
| Euxyl 400 | q.s. |
| Parfümöl, cumarinfrei q.s. | |
| Als Zusatz eignen sich besonders Walnußblätter. | |

| 5. Rezeptur für eine Nachtpflegecreme für fettige, unreine Haut und Mischhaut | |
|---|---|
| Urea pur | 3,0 |
| Ac. lact. | 0,04 |
| Allantoin | 0,2 |
| Sole 25 % | 5,0 |
| Vit. E | 0,2 |
| Vit. B6 | 0,2 |
| Vit. A | 5 000E/g |
| Panthenol | 1,0 |
| Hydroviton | 5,0 |
| Aq. dest. | 10,0 |
| Cremor basalis | 100,0 |
| Euxyl 400 | q.s. |
| Parfümöl, cumarinfrei q.s. | |
| Als Zusatz ist besonders Eichenrinde vorteilhaft. | |

| 6. Rezeptur für ein Kosmetikum zur punktuellen Behandlung umschriebener Hautunreinheiten ausschließlich sogenannter "Pickel". | |
|---|---|
| Vit. A.-Säure | 0,001 |
| Mucilago polyacrylat. cum isoprop. | |
| Cremor basalis | 100,0 |
| Euxyl 400 | q.s. |
| Parfümöl cumarinfrei | q.s. |
| Als Zusatz kommt Aloe vera in Frage. | |

| 7. Rezeptur für eine Tagespflegecreme für trockene und empfindliche Haut | |
|---|---|
| Urea pur | 2,0 |
| Allantoin | 0,2 |
| Ac. lact. | 0,02 |
| Sole 25 % | 3,0 |
| Vit. E | 0,2 |
| Vit. A | 3 000E/g |
| Panthenol | 1,0 |
| Hydroviton | 5,0 |
| Vas. alb. | 20,0 |
| Cremor basalis | 100,0 |
| Euxyl 400 | q.s. |
| Parfümöl, cumarinfrei q.s. | |
| Besonders vorteilhafte Zusätze sind Hesperidin und Acker-Schachtelhalm | |

| 8. Rezeptur für eine Nachtpflegecreme für trockene und empfindliche Haut . | |
|---|---|
| Urea pur | 2,0 |
| Ac. lact. | 0,02 |
| Allantoin | 0,2 |
| Sole 25 % | 3,0 |
| Vit. E | 0,2 |
| Vit. A | 3 OOOE/g |
| Panthenol | 1,0 |
| Hydroviton | 5,0 |
| Vas. alb. | 40,0 |
| Cremor basalis | 100,0 |
| Vorteilhafte Zusätze sind Hesperidin und Ephedrakraut | |

EP 0 351 766 A2

| 9. Rezeptur für eine Feuchtigkeitscreme für normale Haut und für Intermediärpflege für jeden Hauttyp | |
|---|---|
| Urea pur | 2,0 |
| Ac. Lact. | 0,02 |
| Allantoin | 0,2 |
| Sole 25 % | 3,0 |
| Vit. E | 0,2 |
| Vit. A | 3 000E/g |
| Panthenol | 1,0 |
| Hydroviton | 5,0 |
| Aq. dest. | 10,0 |
| Cremor basalis | 100,0 |
| Euxyl 400 | q.s. |
| Parfümöl, cumarinfrei q.s. | |
| Ein vorteilhafter Zusatz ist Brunnenkresse in homöopathischer Verdünnung. | |

| 10. Rezeptur für eine Intensivcreme | |
|---|---|
| Urea pur | 2,0 |
| Ac. lact. | 0,02 |
| Allantoin | 0,2 |
| Sole 25 % | 3,0 |
| Vit. E | 0,2 |
| Vit. A | 3 000E/g |
| Panthenol | 1,0 |
| Hydroviton | 5,0 |
| Vas. Alb. | 5,0 |
| Cremor basalis | 100,0 |
| Euxyl | q.s. |
| Parfümöl, cumarinfrei q.s. | |
| Ein vorteilhafter Zusatz sind Feldstiefmütterchen | |

In allen Rezepturen enthält Euxyl 2-Phenoxy-ethanol und 1,2-Dibrom-2,4-dicyanobutan.

Alle in der vorstehenden Beschreibung erwähnten Merkmale sind als weitere Ausgestaltungen Bestandteile der Erfindung, auch wenn sie nicht besonders hervorgehoben und insbesondere nicht in den Ansprüchen erwähnt sind.

**Ansprüche**

1. Dermatologisches Präparat mit Basiscreme und einem Konservierungsmittel, dadurch gekennzeichnet, daß es natürliche Sole enthält.

2. Präparat nach Anspruch 1, dadurch gekennzeichnet, daß es sich bei der Sole um solche der Solequelle SalzgitterBad handelt.

7

3. Präparat nach Anspruch 1 oder 2, dadurch gekennzeichnet, daß es wenigstens einen weiteren Wirkstoff enthält.

4. Präparat nach Anspruch 3, dadurch gekennzeichnet, daß als weiterer Wirkstoff Panthenol, Harnstoff, Vitamine, Teer, Milchsäure, Collagen oder Schwefel vorgesehen ist.

5. Präparat nach einem der Ansprüche 1 bis 4, dadurch ge kennzeichnet, daß es Euxyl als Konservierungsmittel enthält.

6. Präparat nach einem der Ansprüche 1 bis 5, dadurch gekennzeichnet, daß es wenigstens einen Zusatzemulgator enthält.

7. Präparat nach Anspruch 6, dadurch gekennzeichnet, daß als Zusatzemulgator Genapol, Hostapon oder Texapon vorgesehen ist.

8. Präparat nach einem der Ansprüche 1 bis 7, gekennzeichnet durch wenigstens einen Pflanzenzusatz.

9. Präparat nach einem der Ansprüche 1 bis 8, dadurch gekennzeichnet, daß die Basiscreme eine amphiphile Creme feinster Dispersität ist.

10. Präparat nach einem der Ansprüche 1 bis 9, dadurch gekennzeichnet, daß als Basiscreme die Basiscreme DAC vorgesehen ist.